# EUROPEAN PATENT APPLICATION

(11) **EP 3 708 073 A1**
(43) Date of publication of application: **16.09.2020**
(21) Application number: 20162781.7
(22) Date of filing: 12.03.2020
(51) Int. Cl.: A61B 5/0404, A61B 5/0416, A61B 5/0408, A61B 5/00

(54) **PATIENT MONITORING APPARATUS**

(30) Priority: 14.03.2019 US 201962818271 P
(71) Applicant: Welch Allyn, INC., Skaneateles Falls, NY 13153-0220 (US)
(72) Inventor: BREMER, Edward C, Skaneateles Falls, NY New York 13153-0220 (US); WAWRO, Thaddeus J, Skaneateles Falls, NY New York 13153-0220 (US); SUAREZ, Carlos A, Skaneateles Falls, NY New York 13153-0220 (US); JOVANOVSKI, Brian L, Skaneateles Falls, NY New York 13153-0220 (US); REED, Shelby M, Skaneateles Falls, NY New York 13153-0220 (US); BRICENO-GUTIERREZ, Analia, Skaneateles Falls, NY New York 13153-0220 (US); GRANT, Jennifer M, Skaneateles Falls, NY New York 13153-0220 (US)
(74) Representative: Findlay, Alice Rosemary

(57) **Abstract**

A suite of components for monitoring a subject includes one or more ECG electrodes, a securement component adapted to hold the one or more electrodes to the subject's body, and a host removably connected to or adapted to be removably connected to the one or more electrodes. The host includes a sensor, a processor, and an energy source.

## Description

The subject matter described herein relates to a wearable suite of components for monitoring a subject, such as a medical patient, and to a method of preparing the patient for monitoring by the suite of components.

It is often desirable to monitor selected parameters of a person, referred to herein as a subject or patient. Parameters of interest include vital signs such as respiration rate, and cardiac signals including signals necessary to establish the patient's heart rate. The parameters of interest may also include parameters not traditionally thought of as vital signs such as the patient's position (particularly his position in or relative to a bed) and patient movements including velocity and acceleration. In this specification, references to vital signs or other patient related parameters may refer to the vital sign itself or may refer to the signals detected by sensors, typically electrical signals, processing of which leads to an estimate of the parameter of interest. For example respiration rate may mean the number of breaths per unit time or may mean the sensed signals which can be processed to estimate the number of breaths per unit time. The meaning will be clear from context and/or the distinction between an actual parameter and the signals used to determine its value are interchangable in connection with understanding the following description and the metes and bounds of the accompanying claims.

Parameter monitoring as described above is often carried out in health care settings such as hospitals. Monitoring may also be carried out while the patient goes about his day to day activities. Wearable monitors, which contain the sensors and other components required for the monitoring task, are attractive for such purposes. A wearable monitior is one that is maintained in a fixed position on the patient, for example by being adhered to his skin. "Position" refers to the location of the sensor relative to anatomical features of the patient and may also include the locations or orientations of the sensors relative to each other. "Fixed" means that the position of each sensor remains sufficiently unchanged that its readings remain valid and useful during the time interval that the monitor is in use. In a health care facility a compact, wearable monitor may satisfy monitoring requirements that would otherwise call for the use of multiple pieces of expensive, bulky equipment. In the day to day setting a compact, wearable monitor offers the patient considerable freedom to go about his normal daily activities.

Wearable monitors present a number of design challenges. First, certain of the monitor's sensors must be accurately positioned on the patient in order to achieve the best possible readings. If the patient is to be monitored for a period of, say, several days, it is desirable to leave those sensors in place for the entire duration of the monitoring period. This is because if the sensors are removed and subsequently reapplied to the patient, a caregiver will have to devote time and effort to re-establish accurate positions for the sensors. Even if the caregiver is able to do so, the subsequent location may differ somewhat from the previous location, compromising the repeatability of measuring the signals of interest. In addition, the means used to hold the sensor onto the patient should be breathable in order to allow for air circulation to the patient's skin. In addition, the monitor should be comfortable to wear and durable.

Another design challenge is that some of the monitor's sensors, and/or the electrical components associated with them, might not be tolerant of degrading influences such as radiation exposure during a radiological procedure. Therefore, notwithstanding the desirability of leaving sensors in place for the entire duration of the monitoring period, there may be a need to remove certain sensors from the patient while the degrading influence is present and reapply them to the patient after the degrading influence is no longer present. As noted above it is desirable to re-apply the removed sensors in essentially the same position they occupied relative to the patient's anatomical features prior to removal.

This specification describes a suite of components for monitoring a subject. The suite of components may also be considered to be a kit of components. The suite of components may also be considered to be a system of components, at least when the components are assembled to each other in order to monitor the patient. The component suite includes one or more ECG electrodes, a securement component adapted to hold the one or more electrodes to the subject's body, and a host removably connected to or adapted to be removably connected to the one or more electrodes. The host includes a sensor, a processor and an energy source.

This specification also describes a method of preparing a subject for monitoring with a suite of components which includes 1) one or more electrodes each having a contact portion with an adhesive surface and a snap assembly projecting away from an opposite or top surface of the contact portion, 2) a securement component having an adhesive side and one or more holes with each hole corresponding to a pre-established electrode pattern, and 3) a host adapted to receive and remain removably attached to the electrodes. The method itself includes the steps of A) installing the electrodes in the module thereby forming an electrode/module subassembly, B) placing the subassembly at a monitoring site on the subject whereby the adhesiveness of the adhesive surface of the electrodes causes the electrodes to adhere to the subject, C) disconnecting the module from the adhered electrodes, D) adhering the securement component to the subject so that each snap assembly projects through one of the holes in the securement component, and E) reconnecting the module to the adhered electrodes.

The invention at least in the preferred embodiments provides a means to effect the attachment of a wearable monitor to a patient which has the attributes of breathability, durability, and comfort. The attachment means is resistant to influences that tend to separate the attachment means from the patient and which is resistant to degrading influences. To the extent that the monitor includes sensors that are not resistant to degrading influences, the monitor has an architecture which enables those sensors to be temporarily and conveniently removed, and to be conveniently put back in place after the degrading influence is no longer present.

The invention will now be further described by way of example with reference to the accompanying drawings, in which:
FIG. 1 is an exploded perspective view of a set of ECG electrodes, each of which is a component of a suite of components for monitoring a person, and an electrode release liner.
FIG. 2 is a schematic elevation view of the release liner and electrode elements of FIG. 1.
FIG. 3 is an upward looking plan view in the direction 3--3 of FIG 1.
FIG. 4 is a schematic elevation view of a representative electrode not drawn to the correct scale or proportions.
FIGS. 5 and 6 are plan views of a securement component of the suite of components for monitoring a person showing respectively a patient side and an oppposite or non-patient side and also showing a partially peeled away release liner.
FIG. 7 is an elevation view of the securement component of FIG. 6 drawn with an exaggerated thickness.
FIGS. 8-11 are a top perspective view, a bottom perspective view, a plan view and an elevation view of the exterior of a host component of the suite of components, the host being comprised of a power module and a processor module.
FIG. 12 is a view similar to FIG. 8 in which the housing components of the modules are not illustrated, in order to reveal selected internal elements including a battery cover of the battery module and a printed circuit assembly (PCA) cover of the processsor module, the battery cover and PCA cover being depicted as transparent.
FIG. 13 is an exploded view of the modules of FIGS. 8-11.
FIGS. 14 and 15 are cross sectional side elevation views of the battery module and processor module of FIGS. 8-11 in their assembled states.
FIG 16. is a plan view of a printed circuit assembly suitable for the processor module.
FIGS. 17 and 18 are a plan view and a perspective view showing an ON/OFF switch mounted on a main PCA board and showing the switch in more detail than is discernible in FIG. 16.
FIG. 19 is a cross sectional side elevation view similar to that of FIG. 15 showing the relationship among the host component, the securement component (illustrated with a highly exaggerated thickness) and ECG electrode component(s) when the component suite is being employed to monitor the patient, as represented by the patient's skin.
FIG. 20 is a schematic side elevation view showing a host and a set of electrodes linked to each other by a bridge to show an example of how the set of electrodes may be packaged so that they are spatially distributed in an electrode pattern which matches a connection site pattern of the host.
FIG. 21 is a plan view taken in direction 21--21 of FIG 20.
FIG. 22 is a plan view taken in direction 22--22 of FIG 20.
FIG. 23 is a view of the bridge of FIGS. 20 and 22 shown in isolation, i.e. without electrodes installed in the bridge.
FIG. 24 is a schematic side elevation view showing a set of two electrodes linked together by a flexible bridge.

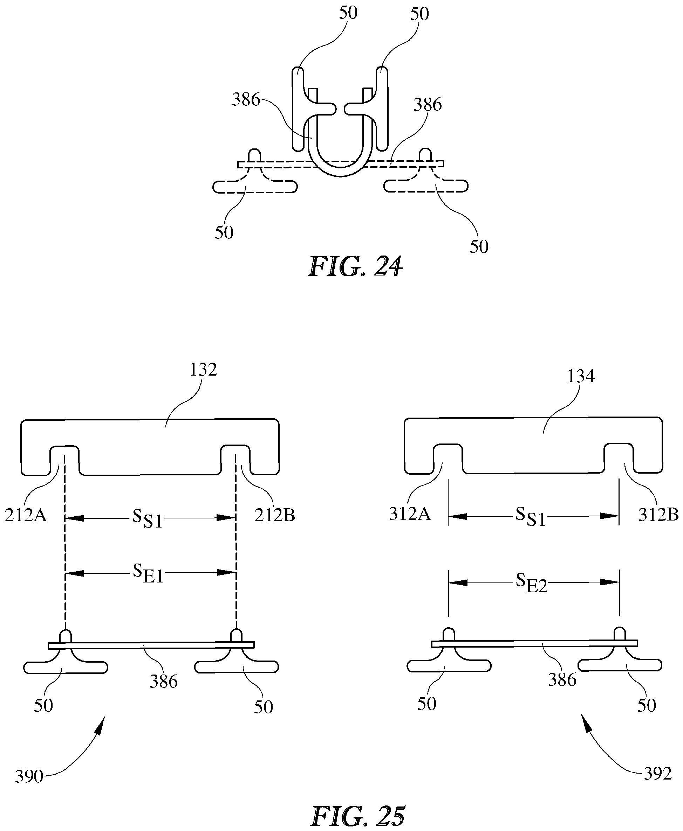

FIG. 25 is a schematic side elevation view showing a specific example of a host comprised of exactly two modules, a first module having exactly two connection sites spaced apart by a first site spacing and a second module having exactly two connection sites spaced apart by a second site spacing, and also showing a first set of exactly two electrodes spaced apart by a first electrode spacing (or constrained to be spaceable from each other by no more than the first electrode spacing), a second set of exactly two electrodes, spaced apart by a second electrode spacing (or constrained to be spaceable from each other by no more than the second electrode spacing), and in which the first connection site spacing equals the first electrode spacing and the second connection site spacing equals the second electrode spacing.
FIG. 26 is a block diagram and a set of associated, schematically illustrated components showing a method of preparing a patient for monitoring with a suite of components which includes one or more electrodes such as ECG electrodes, a securement component, and a host.
FIGS. 27-32 are views illustrating the steps of the block diagram of FIG. 26 in the context of a patient.
FIG. 33 is a block similar to that of FIG. 26 explicitly showing a step of removing a release liner from the electrodes and also showing a schematic illustration of the liner being peeled away from an electrode.
FIG. 34 is a block diagram similar to that of FIG. 26 but including auxiliary steps which reflect the use of a feature for reproducing a test position of the monitor.
FIG. 35 is a view showing an embodiment in which the securement component and two electrodes are part of an assembly in which the securement component and its adhesive extend to a contact portion of each electrode, the assembly being shown in the context of the processor module.
FIG. 36 is a view showing an embodiment of the assembly of FIG. 35 in which the securement component and its adhesive overlie the contact portions of the electrodes and extend to the electrode snap assemblies.

In this specification and drawings, features similar to or the same as features already described may be identified by reference characters or numerals which are the same as or similar to those previously used. Similar elements may be identified by a common reference character or numeral, with suffixes being used to refer to specific occurrences of the element.

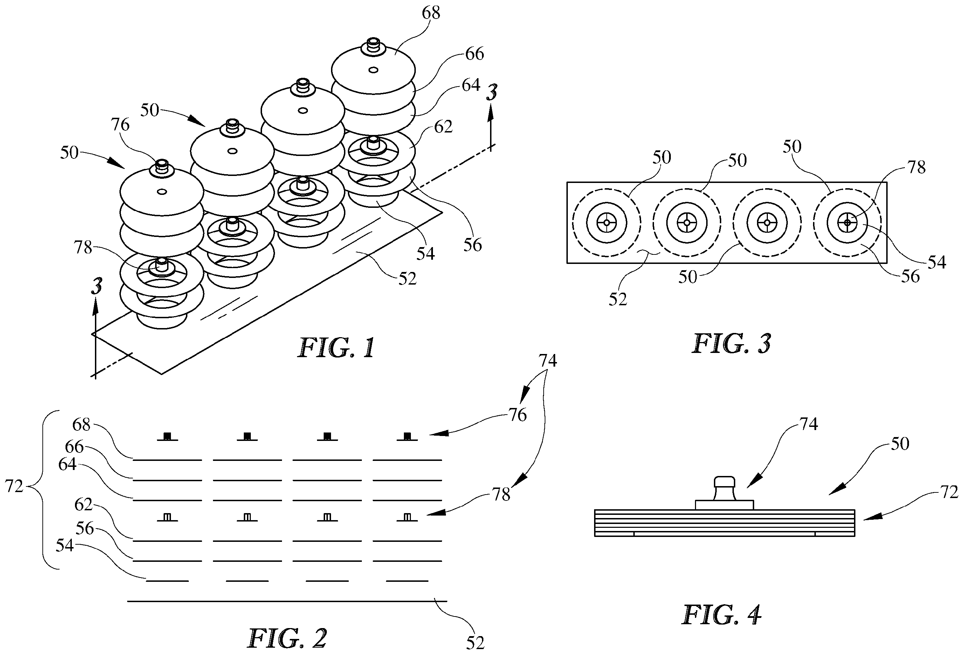

Referring to FIGS. 1-4, a suite of components for monitoring a person, for example a patient, includes one or more electrocardiogram (ECG) electrodes 50. The illustration shows four electrodes mounted on an electrode release liner 52. Arrangements other than that of FIG. 1 are described elsewhere in this specification.

Each electrode 50 includes a patch of comfort gel 54 and a ring of hydrocolloid adhesive 56 whose outer and inner diameters both exceed the diameter of gel patch 54. As a result, dismounting of the electrode from the release liner exposes an annular adhesive surface. Adhesive 56 adheres the electrode to the patient's skin however, as described below, is not the exclusive means for maintaining the electrode at a fixed position on the patient. The comfort gel makes the electrode more comfortable to the patient.

Each electrode also includes a lower pressure sensitive adhesive layer 62, a polyethelene layer 64, an upper foam pressure sensitive adhesive layer 66, and a foam layer 68. Taken collectively, components 54, 56, 62, 64, 66, 68, are referred to as the contact portion 72 of the electrode. Each electrode also includes a snap assembly 74 comprising an electrode magnetic snap 76 and an electrode eyelet 78. When the electrode is in its assembled state, eyelet 78 nests inside magnetic snap 76, and the snap assembly projects above electrode contact portion 72. Despite the nomenclature, magnetic snap 76 of the illustrated embodiment is ferrous, but is not magnetic. As explained in more detail below, the snap assembly and a host component of the component suite are removably connected to each other. However despite the nomenclature "snap", the connection is a magnetic connection, not a mechanical snap connection.

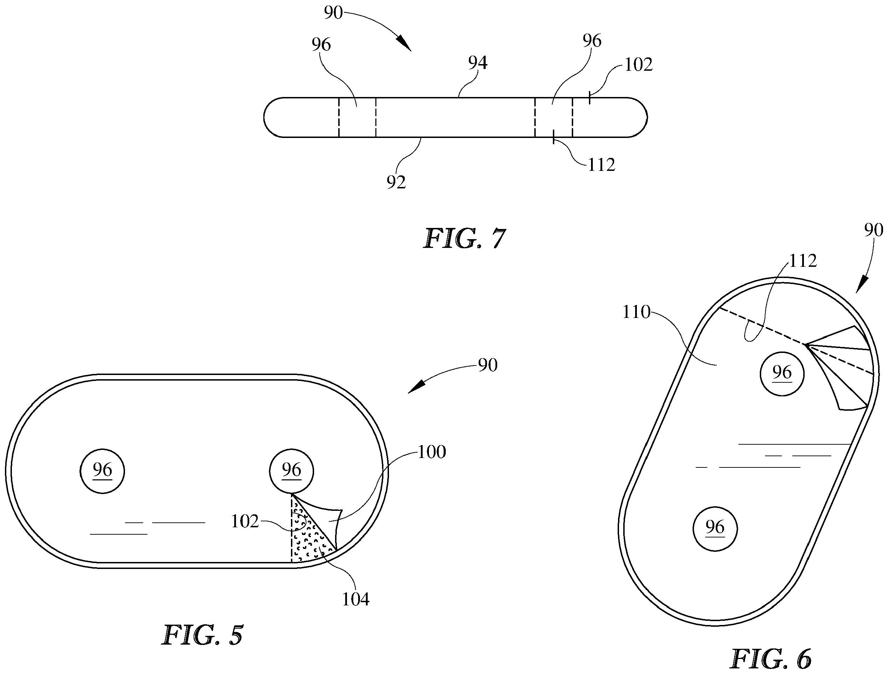

FIGS. 5-7 show a securement component 90 of the suite of components. The securement component is adapted to hold the one or more electrodes 50 to the patient's body. The securement component supplements electrode adhesive 56. The securement component is in the form of a thin pad or patch having a racetrack shaped planform (parallel straight edges joined by first and second arcs) a bottom or patient facing side 92 and a top or non-patient side 94. Two holes 96 penetrate through the securement component. Patient side 92 is covered with a one piece release liner 100 having a perforation 102 that extends from the border of the securement component to one of the holes. As illustrated the release liner has been partially peeled away to reveal an adhesive 104 on the bottom side of the securement component. Hence, the bottom or patient facing side may also be referred to as an adhesive side. Top side 94 is covered with a two piece release liner 110 having a perforation 112 that extends across the width of the securement component. In the elevation view of FIG. 7 the perforations are indicated by vertical marks 102, 112.

The adhesive 104 employed on the bottom of the securement component is one that adheres strongly enough to the patient's skin to resist detachment for a specified period of time, for example approximately seven days, and weakly enough to be intentionally removed from the patient's skin without damaging the patient's skin. One benchmark for skin damage is breaking the skin.

One material which may be useful for the securement component is Tegaderm™ transparent film dressing, a 3M™ product. Tegaderm is said to provide a waterproof, sterile barrier to external contaminants, has an adhesive which is gentle to the skin, and flexes with the skin to enhance patient comfort.

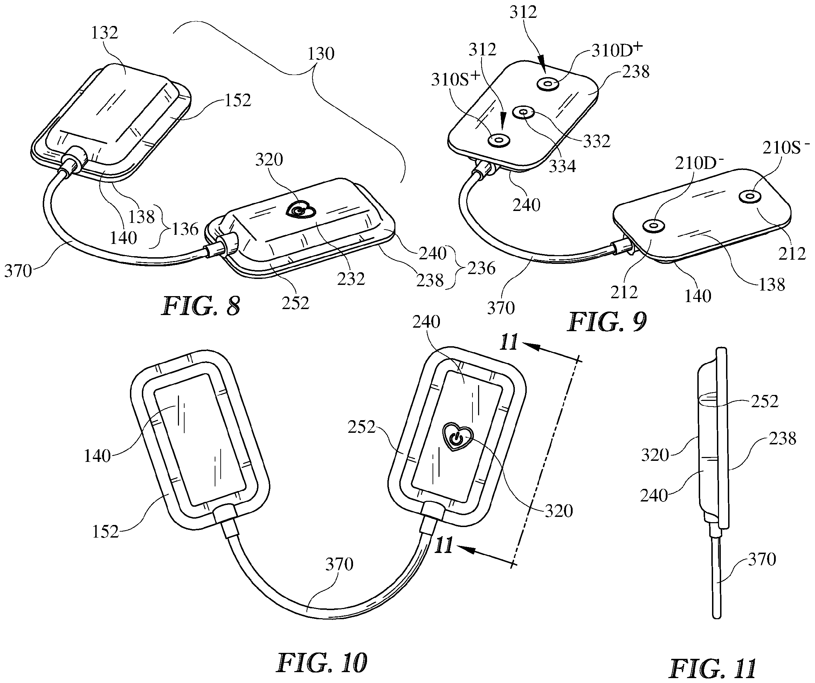

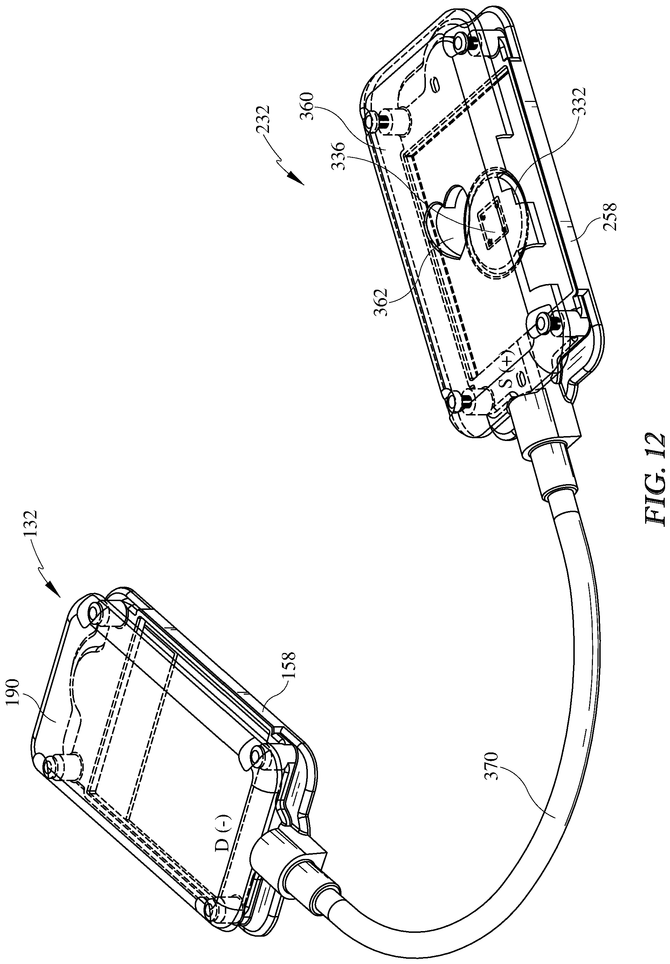

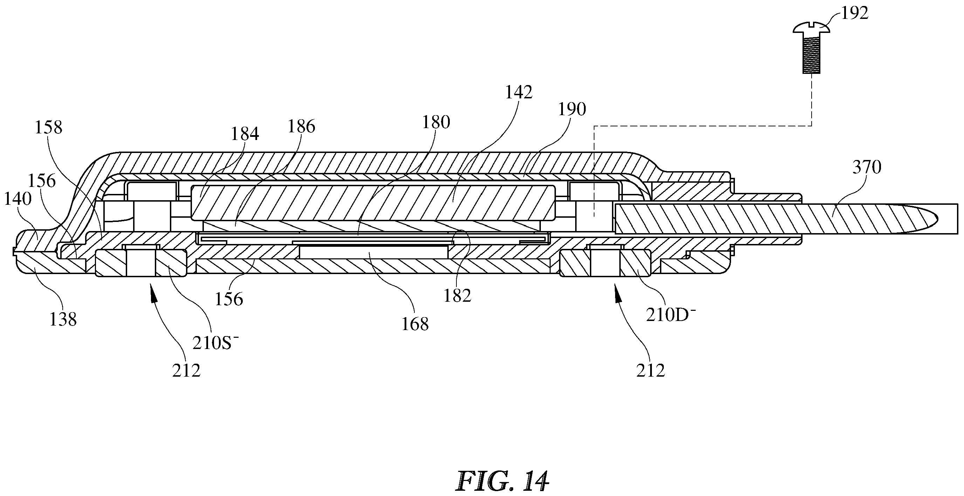

FIGS. 8-15 show a host component 130 of the suite of components. The host is adapted to be removably connected to the one or more electrodes 50 such that the hose receives the electrodes, hence the nomenclature "host". The host includes at least a sensor (other than the ECG electrodes) a processor, and an energy source. As shipped by the manufacturer or distributor, and as initially encountered by an end user, the host is separate from the ECG electrodes. In another embodiment the electrodes are removably pre-installed in the host. As will be appreciated from a complete reading of this specification, the host will be disengaged from the pre-installed electrodes and will later be re-engaged with the electrodes when the component suite is applied to a patient. As used herein, "initially encountered by an end user" means as first seen by the end user who unpackages the components from their shipping container(s). The end user may be a caregiver or, in a "direct to consumer" environment, may be the patient, who serves as his own caregiver. Notwithstanding the foregoing, the phrase "end user" may also apply to a person who unpackages the components on behalf of the patient, caregiver, or patient/caregiver, even though that person is not, strictly speaking, a user of the monitor.

The illustrated host 130 includes a first module 132 which is referred to herein as a power module 132. The power module includes a first housing 136 comprised of a first housing base 138 and a first housing cover 140. The first base and the first cover define an interior 142 of the first housing. Housing base 138 has two coupler openings, 146. The housing cover includes a perimeter flange 152 and an archway 154. A housing adhesive, not shown, on at least the top of the perimeter flange is provided to connect the cover to the base.

The power module also includes a chassis adhesive 156 and a chassis 158. The chassis includes internally threaded posts 166 at each corner thereof, a central opening 168, and an archway seat 170 shaped similarly to archway 154 except for a longitudinally projecting tube segment 172. The bottom of the chassis also includes recesses 174.

The power module also includes a Qi charging receiver 180, a Qi receiver adhesive 182 which holds charging receiver 180 to chassis 158, a battery 184, and a foam battery spacer 186 between the battery and the Qi receiver. The battery is the energy source for the host. Qi charging works by transferring energy from a nearby charger, which is external to the host, to the receiver by way of electromagnetic induction. The charger uses an induction coil to create an alternating electromagnetic field, which the receiver converts back into electricity to be fed into the battery. Wireless charging arrangements other than Qi charging may be used. Wired charging arrangements may also be used.

The power module also includes a battery cover 190. Screws 192 threaded into posts 166 secure the battery cover to the chassis so that battery 184, spacer 186, Qi receiver 180, and Qi receiver adhesive 182 are contained with a space defined by the battery cover and chassis. When cover 140 and base 138 are sealed to each other at cover flange 152, the battery cover 190, battery 184, spacer 186, Qi receiver 180, Qi receiver adhesive 182, chassis 158 and chassis adhesive 156 are all contained in the interior space defined by the base and cover. A band of adhesive 194 between archway 154 and archway seat 170, in combination with the housing adhesive along perimeter flange 152, helps seal against ingress of water and other contaminants into the housing interior.

Magnetic electrode couplers 210S⁻, 210D⁻ reside in chassis recesses 174. "Magnetic", as used in the phrase "magnetic electrode coupler" indicates that the coupler is magnetic. This is in contrast to magnetic snaps 76 of electrodes 50 which, as previously noted, are ferrous but not magnetic. The locations of the couplers are referred to as connection sites and are identified with reference numeral 212. As described in more detail below, when the component suite is being employed to monitor the patient, magnetic attraction between the magnetic couplers 210S⁻, 210D⁻ and snaps 76 of electrode snap assembly 74 cause power module 132 to be removably engaged with the ECG electrodes. The couplers also serve as impedance plethysmography (IPG) negative drive and sense electrodes, hence the D⁻ and S⁻ suffixes. The unsuffixed reference numeral 210 may be used herein when the text is referring to the coupling function of the couplers but not to their plethysmography function.

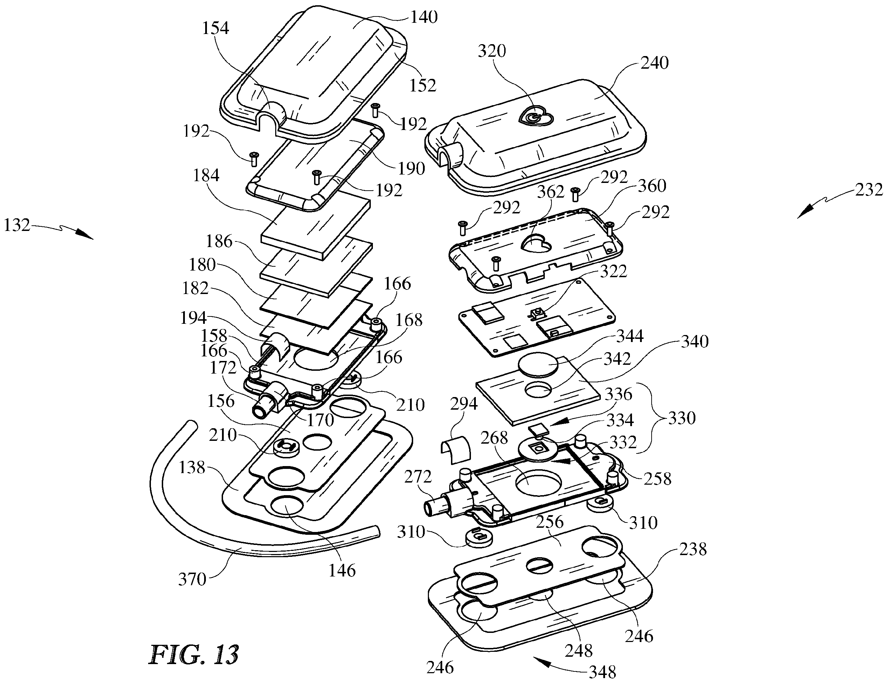

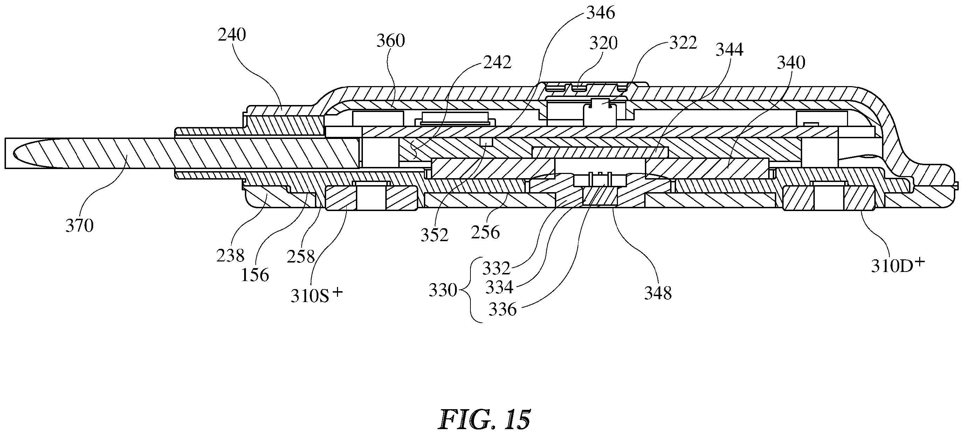

The illustrated host also includes a second module 232 which is referred to herein as a processor module 232. Features of the processor module which are the same as or analogous to features of the power module are or may be identified with the reference numerals of the power module incremented by 100. The processor module chassis adhesive 256, chassis 258, magnetic couplers 310S⁺, 310D⁺ (analogous to couplers 210S⁻, 210D⁻ of power module 132), and adhesive band 294 are the same as their counterpart elements of the power module. (Unsuffixed reference numeral 310 may be used herein when the text is referring to the coupling function of the couplers but not to their plethysmography function.) The processor module housing cover 240 is the same as the power module housing cover 140 except that the processor module has a heart shaped emblem 320, and the power module is either plain as in FIG. 13 or includes instructions and/or other information and markings, depicted generally as feature 220, as seen best in FIGS. 8 and 13. The processor module housing base 238 is the same as the power module housing base 138 except that housing base 238 includes a center opening 248. The visible difference in the covers, particularly the heart shaped emblem, remind the caregiver of the correct polarity of the modules, namely that the power module should be positioned to the right of the patient's medial anatomical plane and the processor module should be situated to the left of the medial plane. The heart shaped emblem also reveals the location of an ON/OFF switch 322, which is below the emblem as seen best in FIGS. 13 and 15. In at least the vicinity of hert shaped emblem 320, cover 240 is pliable enough that a person can press down on the cover to actuate the ON/OFF switch.

The processor module also includes a temperature sensing assembly 330 comprised of a sensor housing 332, a temperature sensor 334 and temperature sensor circuitry 336. The illustrated temperature sensor is an infrared sensor, i.e. it is responsve to infrared wavelengths. The illustrated sensor circuitry is a printed circuit assembly (PCA). As seen best in FIG. 15, the temperature sensing assembly occupies the central opening 268 of the chassis and the center opening 248 of the housing base. An infrared transparent window 348 covers opening 248 to resist ingress of contaminants.

The processor module also includes a foam spacer layer 340 with a center hole 342, and a main circuit board or main PCA 346. Wires, not shown, pass through center hole 342. A foam plug 344 also occupies the hole. Together, foam layer 340 and plug 344 provide thermal and electrical insulation between main PCA 346 and temperature sensor 334.

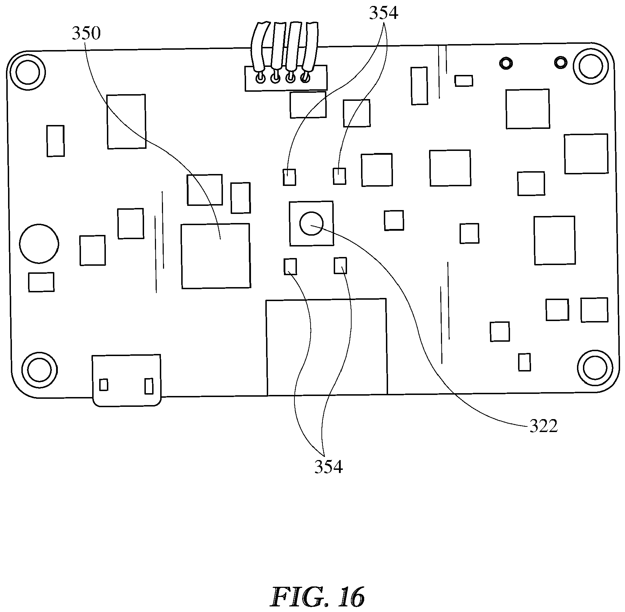

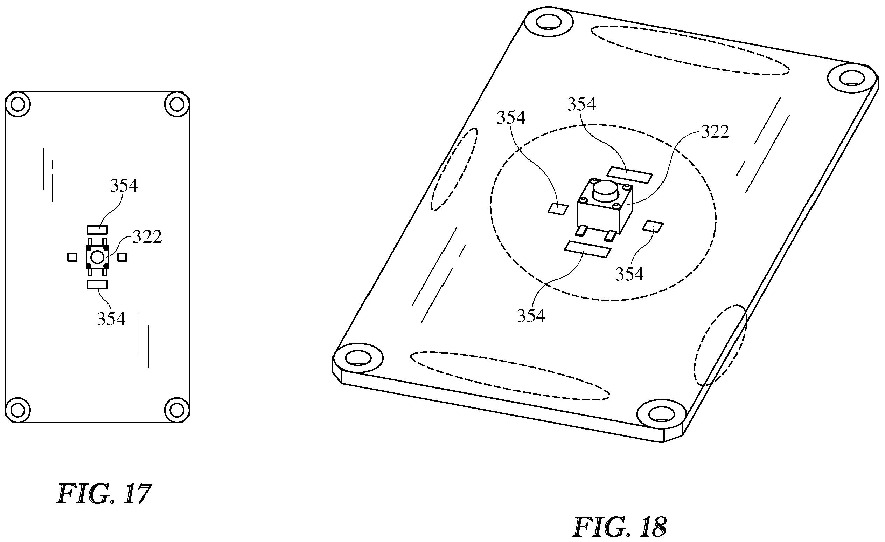

Referring additionally to FIGS. 16 and 17-18, the main PCA includes a processor 350, an ON/OFF switch 322, an accelerometer 352, (FIG. 15), Qi charging circuitry (not illustrated), and the connections to the IPG drive and sense electrodes 210S⁻, 210D⁻, 310S⁺, 310D⁺. The PCA also includes one or more light emitting diodes (LED's) 354. Light emitted by the LED's can be seen through cover 240 of processor module 232. The LED's may be used to indicate whether the monitor is ON or OFF and may also be used to indicate information other than ON/OFF. The PCA also includes circuitry for generating IPG drive signals and for processing the signals from the IPG sense electrodes. The PCA also includes circuitry for extracting information from the ECG electrodes.

Processor module 232 also includes a PCA cover 360 with a heart shaped opening 362. When the module is assembled as seen in FIGS. 8-11, heart shaped opening 362 registers with heart shaped emblem 320 on housing cover 240. The processor module may also include a wireless radio circuitry (not illustrated) to convey parameter signals of interest to an off-board receiver.

In summary, the processor module includes at least one sensor. These may include IPG sensors for estimating the patient's respiratory rate, temperature sensor 334 for estimating the patient's skin temperature, and an acceleration sensor 352. Readings from the accelerometer may be used to monitor for accelerations that might indicate a health related problem (e.g. tremors, shivering, coughing) or might indicate that the patient in moving in an undesirable way (e.g. attempting to make an unauthorized exit from his bed). The accelerometer readings may also be integrated or double integrated over time to establish an estimate of the speed of patient movement and the patient's present position.

Host 130 also includes a conduit 370. Each end of the conduit circumscribes one of the tube segments 172, 272 that extend from the chassis of the power and processor modules. An adhesive, not shown is applied to the outside surface of the tube segments to help secure the connection. The tubes may also be barbed to resist separation of the conduit. The conduit has a length such that when the ECG electrodes are correctly positioned on the patient and engaged with modules 132, 232, the conduit will accommodate a patient size range from a 5th percentile female to a 95th percentile male. Wiring, not shown, extends through the conduit. The wiring includes two wires from the battery, two wires connecting the Qi charger to the Qi charger circuitry, one wire from the IPG sense electrode and one wire from the IPG drive electrode.

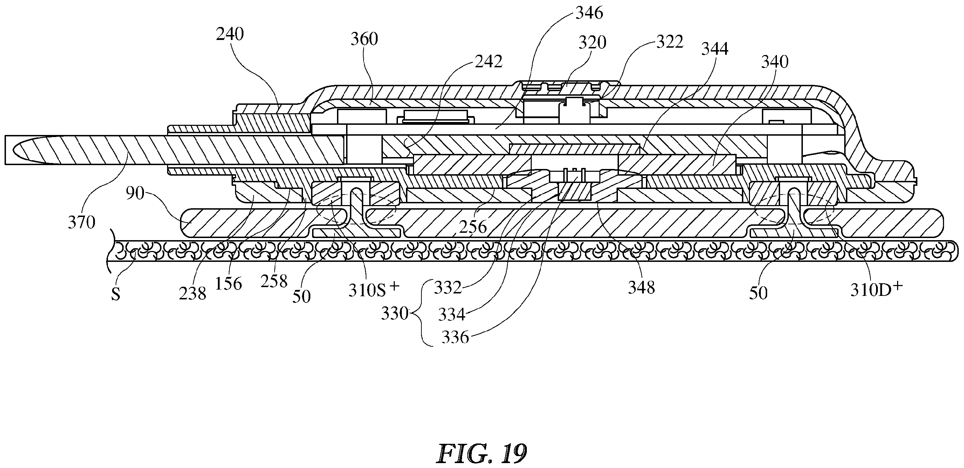

FIG. 19 is a cross sectional side elevation view showing the relationship among the host component 130 (represented by power module 132), the securement component 90 and ECG electrode component(s) 50 when the component suite is being employed to monitor the patient. The ECG electrodes 50 adhere to the patient's skin S by way of the ring of hydrocolloid adhesive 56 (FIG. 1). In the illustration of FIG. 19 securement component release liners 100, 110 have been previously removed so that the adhesive 104 on the bottom side of the securement layer is exposed. A first portion 380 of the securement component (indicated by the dashed borders) overlies the contact portions of the electrodes. A second portion 382 of the securement component sticks to the patient's skin. Thus, as previously noted, the securement component supplements the electrode hydrocolloid adhesive 56. The adhesive 104 of the securement component helps provide a watertight seal around the perimeters of the ECG electrodes. The electrode snap assemblies 74 project through holes 96 in the securement component.

Host 130, represented in FIG. 19 by processor module 232, is removably connected to the ECG electrodes so that securement component 90 resides between the patient and base 238 of the module. The connection is effected by the attraction of the magnetic snap 76 of the electrodes to the magnetized magnetic coupler 310 of the module. The connection is both a mechanical connection and an electrical connection between the host and the electrodes. The electrical connection transports signals from the ECG electrodes. Power module 132 is similarly removably engaged with other ECG electrodes by the magnetic attraction between the magnetic snaps of those electrodes to power module magnetic couplers 210. If desired, the mechanical connection could be made by mechanical snaps or some other nonmagnetic arrangement.

Continuing to refer to FIG. 19, the securement component adheres strongly to the patient's skin and resists influences that tend to separate it from the patient's skin. The ECG electrodes are therefore held in place. However the securement component adheres weakly enough to be intentionally removed from the patient's skin without damaging the skin.

The magnetic attraction between electrode snaps 76 and housing magnetic couplers 210, 310 is strong enough to hold the module and its sensors in a fixed position relative to the ECG electrodes, and therefore in a fixed position relative to the patient's anatomical features. The magnetic attraction is strong enough to achieve this state of securement even if the module is bumped or otherwise exposed to actions or conditions that might dislodge it from the electrodes. However the magnetic attraction is weak enough that if the module needs to temporarily removed, such removal can be easily accomplished without special tools and with a force well within the capabilities of a human caregiver. As already explained such temporary removal might be required to avoid subjecting the module, or the components contained within it, to a degrading influence such as radiation. Moreover, the required separation force is also small enough that its application will break the magnetic bond at the snap/coupler interface rather than separating the securement component from the patient's skin.

In view of the foregoing, certain variations and generalizations can now be better appreciated.

In general, the host can be a single module or can be comprised of three or more modules, rather than the two modules described so far. Each module may include fewer or more than the two connection sites described above. Moreover the quantity of connection sites need not be the same on all the modules. If the quantity of connection sites is the same on two or more modules, is it not necessary for the spatial distribution pattern of those connection sites to be the same.

In a more specific example in which the host is comprised of exactly two modules, a first of the two modules is adapted to be removably connected to M ECG electrodes. The first module includes exactly M electrode connection sites, one for each of the M ECG electrodes. The M electrode connection sites are spatially distributed in a first connection site pattern. "Connection site pattern" means the spatial relationships amongst the M connection sites, A second module is adapted to be removably connected to N ECG electrodes. The second module includes exactly N electrode connection sites, one for each of the N ECG electrodes. The N electrode connection sites are spatially distributed in a second connection site pattern. The set of electrodes includes exactly M + N electrodes. In one specific embodiment M = N.

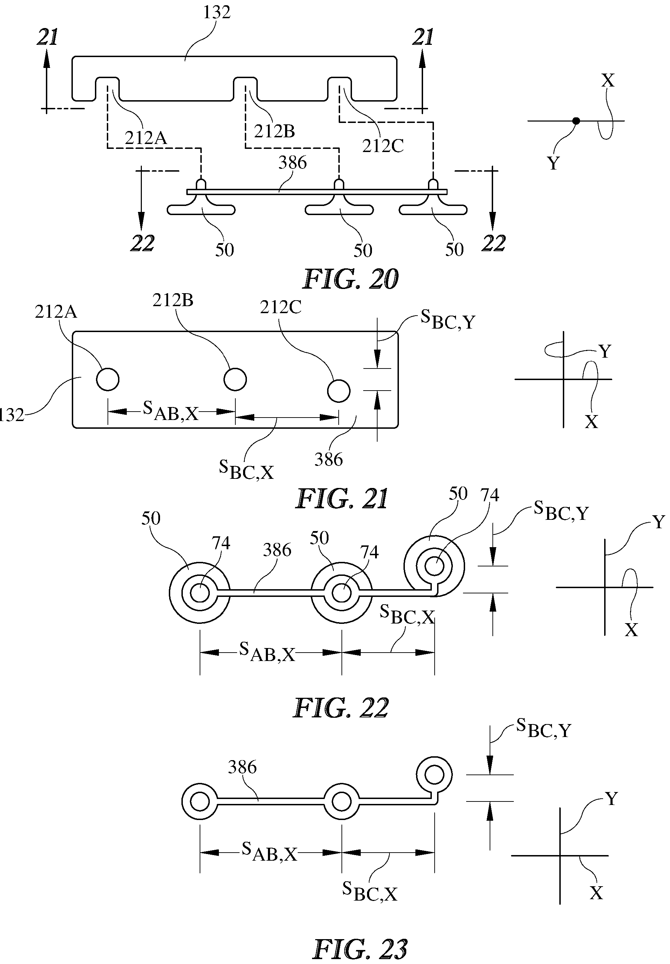

Referring to FIGS 20-23, the set of M electrodes may be packaged so that they are spatially distributed in a first electrode pattern which matches the first connection site pattern. FIGS. 20-22 shows an example in which M = 3. The electrode connection sites on the first module (e.g. power module 132) are arranged in a first connection site pattern in which site 212B is spaced from site 212A by a spacing S_{AB,X} in the X direction, and site 212C is spaced from site 212B by a spacing S_{BC,X} in the X direction and by a spacing S_{BC,Y} in the Y direction. A bridge 386 packages electrodes 212A, 212B, 212C together so that the electrodes are arranged in a first electrode pattern which matches the first connection site pattern. The user can insert the electrode snap assemblies into the electrode couplers at the connection sites as a unit, rather than having to deal with each electrode individually. When the bridge is no longer needed, or when its presence is no longer of value, it can be released from the electrodes and discarded or recycled.

The set of N electrodes may be packaged similarly to the way just described in connection with the set of M electrodes so that they are spatially distributed in a second electrode pattern which matches the second connection site pattern. The above example of the M electrode arrangement suffices for the reader to understand the N electrode arrangement. The first and second connection site patterns may be replicas of each other or may differ from each other. M may be equal to N or M may differ from N. In one embodiment M = N = 2.

As seen in FIGS. 20, 22 and 23 the bridge is rigid so that the electrodes are distributed in the desired electrode pattern. FIG. 24 shows an alternative in which the bridge is nonrigid (in the illustrated example M (or N) equals two). Therefore the ECG electrodes, as initially encountered by an end user, may not be spatially distributed in an electrode pattern that matches the host connection site pattern (solid lines). However because the bridge can be unfolded (dashed lines) the electrodes are distributable in an electrode pattern that matches the host connection site pattern.

FIG. 25 shows a specific example in which host 130 includes exactly two modules, first module 132 and second module 232. The first module includes exactly two connection sites 212A, 212B spaced apart by a first site spacing S_{S1}. The second module also includes exactly two connection sites 312A, 312B spaced apart by a second site spacing S_{S2}. The one or more electrodes comprises a first set 390 of exactly two electrodes and a second set 392 of exactly two electrodes. The electrodes of the first set are spaced from each other by a first electrode spacing S_{E1} (or are constrained to be spaceable from each other by no more than the first electrode spacing S_{E1}, for example by using a nonrigid bridge as explained above). Spacing S_{E1} equals spacing S_{S1}. The electrodes of the second set are spaced from each other by a second electrode spacing S_{E2} (or are constrained to be spaceable from each other by no more than the second electrode spacing S_{E2}). Spacing S_{E2} equals spacing S_{S2}.

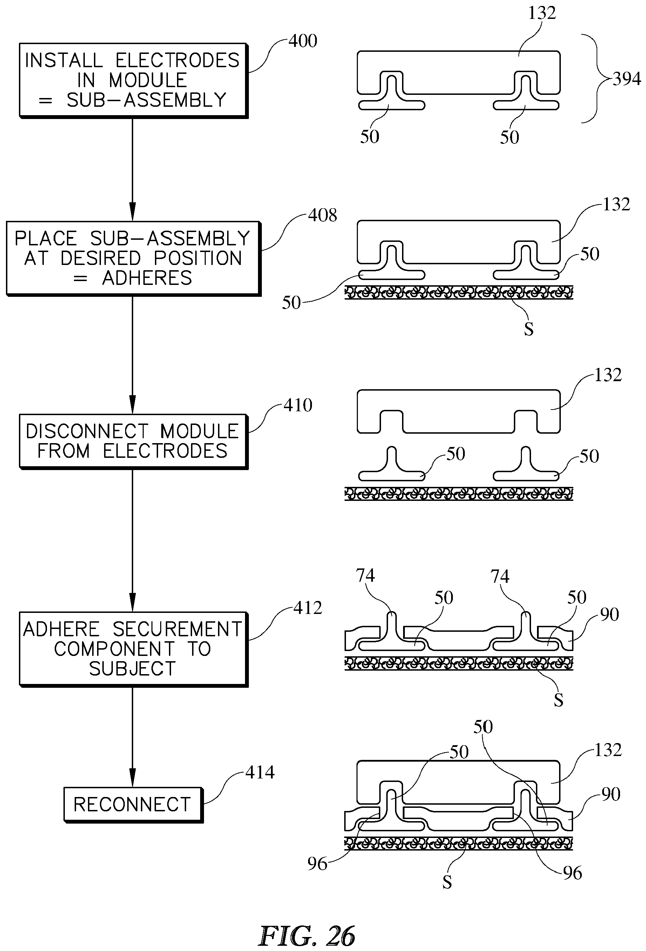

FIG. 26 is a block diagram showing a method of preparing a patient for monitoring with a suite of components which includes 1) one or more electrodes each having a contact portion with an adhesive surface and a snap assembly projecting away from an opposite surface of the contact portion, 2) a securement component having an adhesive side and one or more holes, each of which corresponds to a pre-established electrode pattern, and 3) a host adapted to receive and be removably attached to the electrodes. The host is represented by a single module, for example power module 132 or processor module 134. FIG. 26 also includes a hardware schematic next to each block to show the hardware configuration that results from the performance of each step of the method.

The method includes the step, at block 400, of installing the electrodes 50 in the module 132 thereby forming an electrode/module subassembly 394.

The method also includes the step, at block 408, of placing subassembly 394 at a monitoring site on a patient. The adhesiveness of the adhesive surface causes the electrodes to adhere to the patient, e.g. to his skin.

The method also includes the step, at block 410, of disconnecting the module from the adhered electrodes. In other words a caregiver lifts up the module, breaking the magnetic connection between the snap assemblies of the electrodes and the magnetic coupler of the host. The adhesiveness of the adhesive surface causes the electrodes to remain adhered to the patient.

The method also includes the step, at block 412, of adhering the securement component to the patient so that each snap assembly 74 projects through one of holes 96 in the securement component.

The method also includes the step, at block 414, of reconnecting the module to the adhered electrodes. In other words the caregiver places the module over the electrodes so that the magnetic connection between each electrode and its companion magnetic coupler is re-established.

At the conclusion of reconnecting step 414 the monitor is secured to the patient, with the module removably secured to the electrodes. Securement of the electrodes to the patient is enhanced by the presence of securement layer 90. The caregiver may then press the ON/OFF switch on the module to commence patient monitoring.

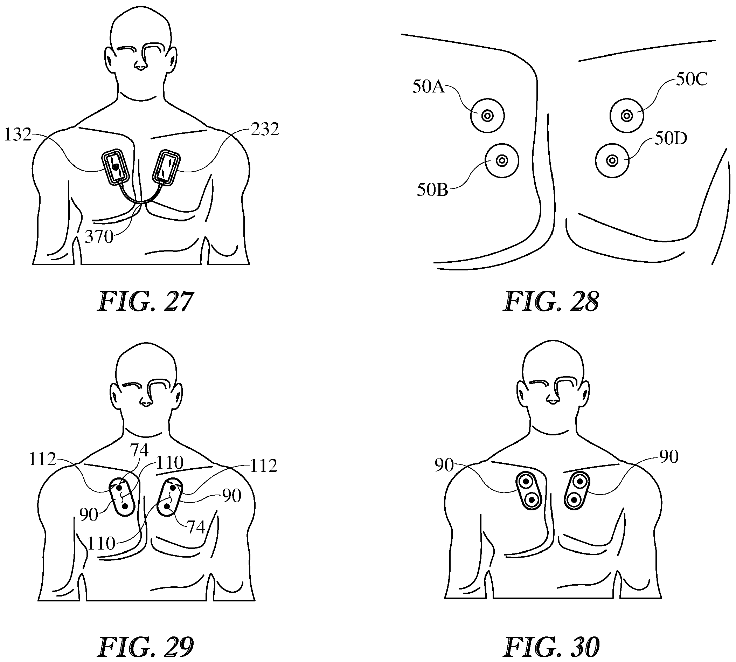

FIGS. 27-33 are views illustrating the steps of the block diagram of FIG. 26 in the context of a patient.

FIG. 27 shows the module/electrode subassemblies placed against the patient's chest at a selected monitoring site, corresponding to block diagram block 408. FIG. 28 shows that after the modules have been disengaged from the electrodes, the electrodes remain adhered to the patient. FIG. 28 corresponds to block diagram block 410. The four electrodes define a single lead ECG arrangement. Electrode 50A, 50B, 50C, 50D mate respectively with with magnetic couplers (IDG sense and drive electrodes) 210S-, 210D-, 310D+, 310S+. The electrodes are placed symmetrically relative to the patient's sternum. The uppermost electrode of the left electrode pair is approximately laterally aligned with the middle of the patient's left clavicle. The uppermost electrode of the right electrode pair is approximately laterally aligned with the middle of the patient's right clavicle. Electrode 50A is placed at about the second right intercostal space. Electrode 50D is placed at about the fourth left intercostal space.

FIG. 29 shows the securement component, with adhesive side release liner 100 having been removed, adhering to the patient so that each snap assembly 74 projects through one of the holes in the securement component. The release liner 110 on the patient side 92 of the securement component is still in place. In FIG. 30 the top side release liner 110 has been removed. FIGS. 29-30 correspond to block diagram block 412.

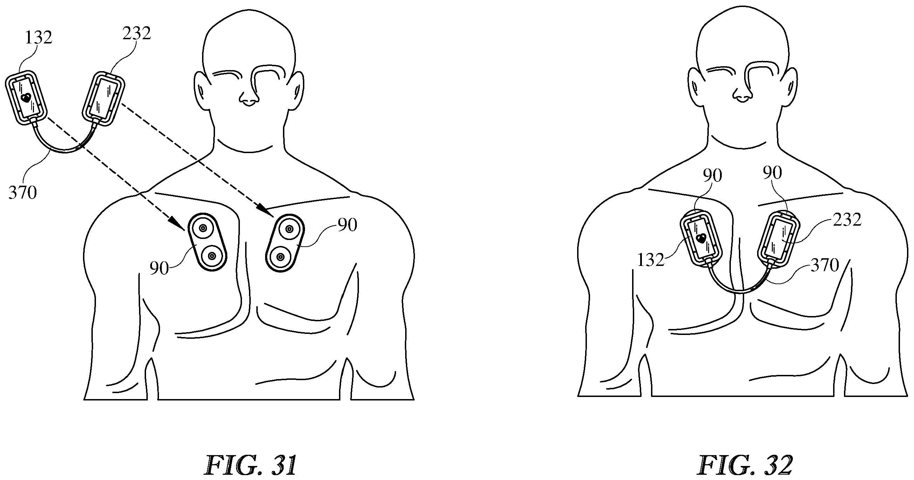

FIGS. 31-32 show re-engagement of the power and processor modules with the ECG electrodes so that the magnetic connection between each electrode and its companion magnetic coupler is re-established. FIGS. 30-31 correspond to block diagram block 414.

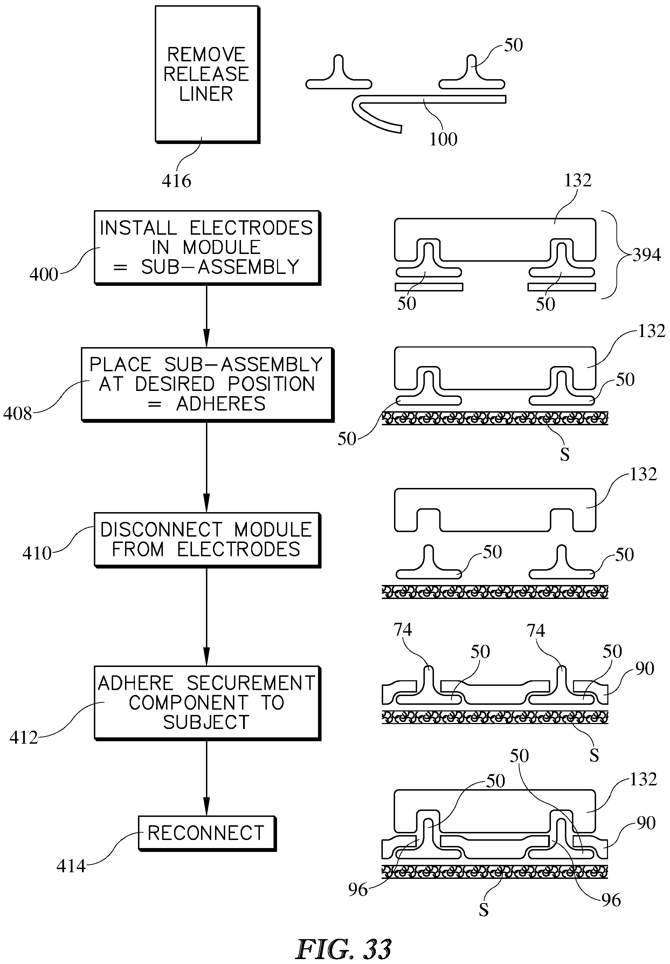

The method shown in FIG. 26 presumes that the electrode release liner has been peeled off the electrodes prior to the step at block 408. FIG. 33 shows this explicitly at block 416 and the associated schematic. Block 416 is separate from the method sequence at blocks 400, 408, 410, 412, 414, and is vertically longer than block 400 to show that the release liner can be removed at any time before step 408. As a practical matter it may be best to not remove the release liner until after step 400 has been accomplished. That way the hydrocolloid adhesive will not be exposed while the caregiver is installing the electrodes in the module.

As noted in the Background section of this application, certain of the monitor's sensors must be accurately positioned on the patient in order to achieve the best possible readings. In other words the caregiver needs to identify a location on the patient's body that will be clinically satisfactory for the monitor. To do so the caregiver can position the monitor on or near the patient's body with patient side release liner 100 still covering the adhesive layer 56 of the electrodes. However when the caregiver lifts the monitor away from the patient in order to peel off the protective liner, he may lose track of the identified satisfactory location.

Therefore, the monitor may include one or more features to address the above described difficulty. Examples of such features are the progressive release feature illustrated in FIGS. 25-26 of US Patent Application 16/150,572, and registration notches illustrated in FIGS. 27-28 of that application.

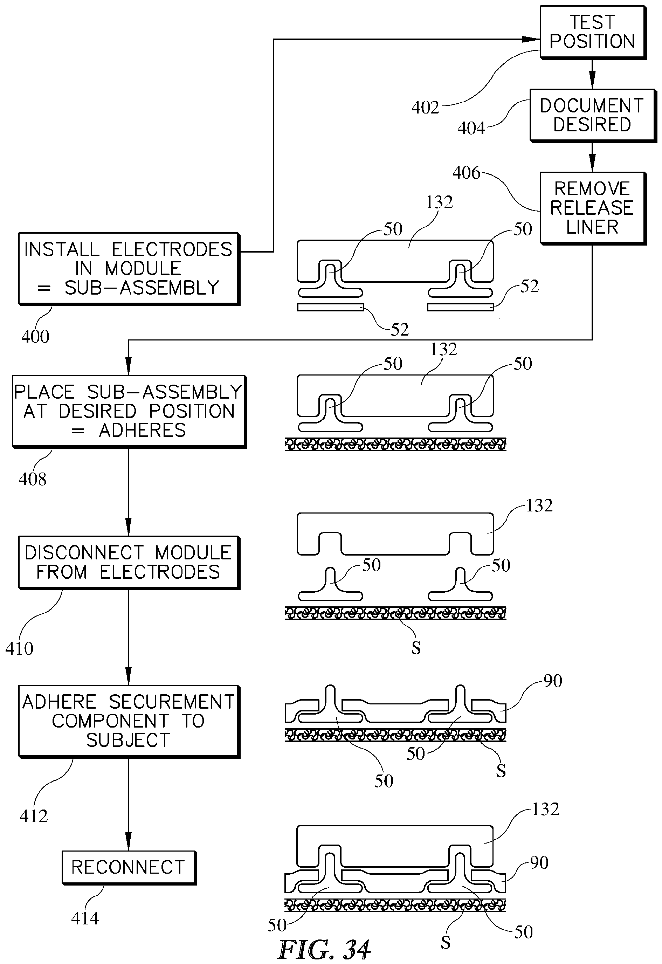

FIG. 34 is a block diagram similar to that of FIG. 26, but including auxiliary steps which reflect the use of the progressive release feature, registration notches, or some other feature provided to address the same problem. Step 400 is carried out with the electrode release liner in place. The method then branches to test positioning step 402. Once the caregiver determines a satisfactory position, the method advances to step 404 where the caregiver documents the desired location. At block 406 the caregiver removes the electrode release liner. The method then advances to step 408. Beginning at step 408 the method proceeds as already described in connection with FIG. 26.

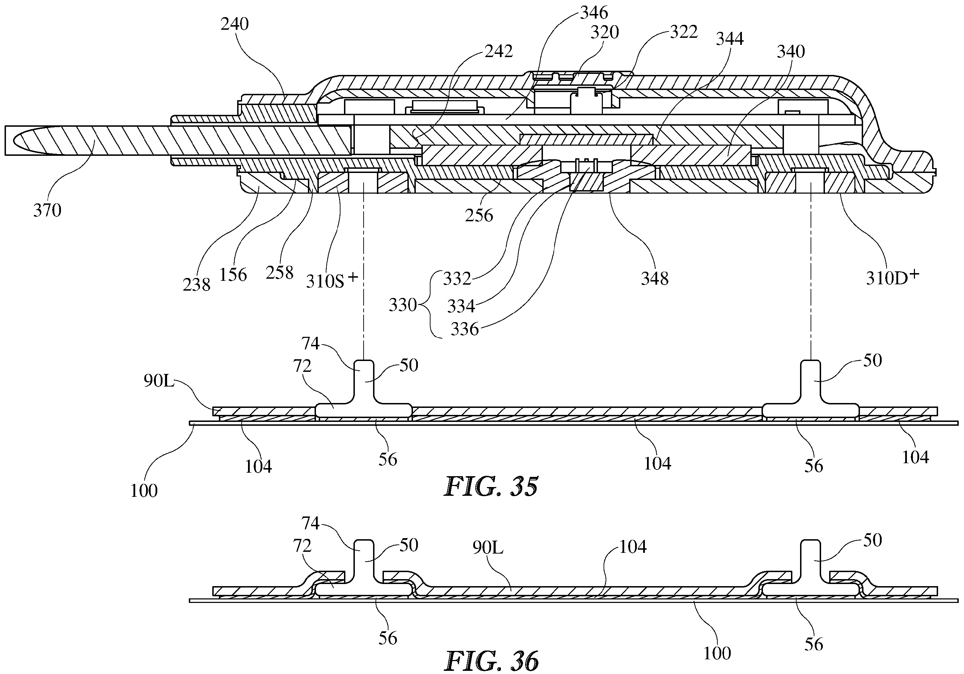

In the foregoing description, securement component 90 is described as a component separate from the host component 130 (power module 132 and processor module 232) and from EKG electrodes 50. In another embodiment the securement component is part of an assembly that includes an electrode or a set of electrodes. FIG. 35 shows an example in which the securement component 90 is a layer 90L of an assembly comprised of two electrodes 50, securement component 90 with adhesive 104, and release liner 100. The securement component 90 and its adhesive 104 extend to contact portion 72 of each electrode. FIG. 36 shows another variant of the assembly in which securement component 90 and its adhesive 104 overlie the contact portions of the electrodes and extend to the electrode snap assemblies 74.

Although this disclosure refers to specific embodiments, it will be understood by those skilled in the art that various changes in form and detail may be made.

Embodiments of the invention can be described with reference to the following numbered clauses, with preferred features laid out in the dependent clauses:
1. A suite of components for monitoring a subject, comprising:
   one or more ECG electrodes;
   a securement component adapted to hold the one or more electrodes to the subject's body; and
   a host removably connected to or adapted to be removably connected to the one or more electrodes, the host including a sensor, a processor and an energy source.
2. The suite of components of clause 1 wherein the host also includes radio circuitry.
3. The suite of components of either clause 1 or clause 2 wherein the host comprises a power module which includes the energy source and a processor module which includes the processor.
4. The suite of components of clause 3 wherein the processor module also includes the sensor.
5. The suite of components of any preceding clause wherein the energy source is a battery and the power module includes a charging receiver.
6. The suite of components of clause 5 wherein the charging receiver is a Qi charger.
7. The suite of components of any preceding clause wherein the sensor is at least one of:
   a) an impedance plethysmography (IPG) receive sensor;
   b) a temperature sensor; and
   c) an accelerometer.
8. The suite of components of any preceding clause wherein the host comprises:
   a power module which includes a first housing comprised of a first housing base and a first housing cover, the first base and the first cover defining an interior of the first housing, and wherein the energy source is a battery residing in the interior space;
      and
   a processor module which includes a second housing comprised of a second housing base and a second housing cover, the second base and the second cover defining an interior of the second housing, the interior of the second housing containing:
      a) a main circuit assembly,
      b) an ON/OFF switch,
      c) an accelerometer, and
      d) a temperature sensing assembly.
9. The suite of components of clause 8 in which the power module and the processor module each include an electrode coupler whereby the power module can be coupled to at least one first ECG electrode by way of the power module electrode coupler(s) and the processor module can be coupled to at least one second ECG electrode by way of the processor module electrode coupler(s).
10. The suite of components of clause 9 wherein the power module electrode couplers include a first IPG drive electrode and a first IPG sense electrode, and the processor module electrode couplers include a second IPG drive electrode and a second IPG sense electrode.
11. The suite of components of any one of clauses 8 to 10 comprising a conduit having wiring extending therethrough for conveying electrical energy from the battery to the processor module to power components of the processor module.
12. The suite of components of clause 11 having additional wiring extending therethrough for conveying information between the battery module and the processing module.
13. The suite of components of any one of clauses 8 to 12 wherein the temperature sensing assembly includes a temperature sensor and a temperature sensor circuit.
14. The suite of components of clause 13 wherein the temperature sensor is responsive to infrared wavelengths.
15. The suite of components of any preceding clause wherein:
   a) each electrode includes a snap assembly; and
   b) the securement component is a pad having a bottom side and a top side, the bottom side including an adhesive, the pad also including a hole through which the snap assembly projects when the suite of components is being employed to monitor the subject.
16. The suite of components of clause 15 wherein the adhesive is one that adheres strongly enough to the subject's skin to resist detachment for approximately seven days and weakly enough to be intentionally removed from the subject's skin without damaging the subject's skin.
17. The suite of components of any preceding clause wherein:
   a) each electrode includes a contact portion and a snap assembly extending from the contact portion;
   b) the securement component is a pad having a bottom side and a top side, the bottom side including an adhesive, the pad also including a hole therethrough;
   c) the host includes a base; and
   d) when the suite of components is arranged to monitor the subject, the securement component resides between the base and the subject, a first portion of the securement component overlies the contact portion of the electrode, a second portion of the securement component sticks to the subject's skin, and the snap assembly projects into the host.
18. The suite of components of any preceding clause wherein the securement component has a racetrack planform.
19. The suite of components of any preceding clause wherein, as initially encountered by an end user thereof, the host is separate from the one or more ECG electrodes.
20. The suite of components of clause 19 wherein:
   the host includes exactly two modules as set forth below:
      a first module adapted to be removably connected to M ECG electrodes, the first module including exactly M connection sites spatially distributed in a first connection site pattern and
      a second module adapted to be removably connected to N ECG electrodes, the second module including exactly N connection sites spatially distributed in a second connection site pattern; and
   the one or more ECG electrodes includes exactly N + M elecrodes.
21. The suite of components of either clause 19 or clause 20 wherein the one or more electrodes includes:
   a first set of M electrodes which are spatially distributed or spatially distributable in a first electrode pattern which matches the first connection site pattern; and
   a second set of N electrodes which are spatially distributed or spatially distributable in a second electrode pattern which matches the second connection site pattern.
22. The suite of components of clause 21 wherein N = M = 2 and the second connection site pattern is a replica of the first connection site pattern.
23. The suite of components of any one of clauses 19 to 22 wherein:
   the host includes exactly two modules as set forth below:
      a first module which includes exactly two connection sites spaced apart by a first site spacing Ssi; and
      a second module which includes exactly two connection sites spaced apart by a second site spacing S_{S2}; and
   the one or more electrodes comprise:
      a first set of exactly two electrodes which are spaced from each other by a first electrode spacing S_{E1} or are constrained to be spaceable from each other by no more than the first electrode spacing S_{E1} wherein S_{E1} = Ssi; and
      a second set of exactly two electrodes which are spaced from each other by a second electrode spacing S_{E2} or are constrained to be spaceable from each other by no more than the second electrode spacing S_{E2} wherein S_{E2} = S_{S2}.
24. a method of preparing a subject for monitoring with a suite of components which includes 1) one or more electrodes each having a contact portion with an adhesive surface and a snap assembly projecting away from an opposite surface of the contact portion, 2) a securement component having an adhesive side and one or more holes, each hole corresponding to a pre-established electrode pattern, and 3) a host adapted to receive and be removably attached to the electrodes, the method including the steps of:
   A) installing the electrodes in the module thereby forming an electrode/module subassembly;
   B) placing the subassembly at a monitoring site on the subject whereby the adhesiveness of the adhesive surface causes the electrodes to adhere to the subject;
   C) disconnecting the module from the adhered electrodes;
   D) adhering the securement component to the subject so that each snap assembly projects through one of the holes in the securement component; and
   E) reconnecting the module to the adhered electrodes.
25. The method of clause 24 wherein the adhesive surface of the contact portion is covered by an electrode release liner and the method includes the step, carried out prior to step (B), of removing the electrode release liner from each electrode.
26. The method of either clause 24 or clause 25 wherein the adhesive surface of the contact portion is covered by an electrode release liner and the method includes:
   a) leaving the release liner in place during step (A);
   b) carrying out the steps set forth below prior to carrying out step B:
      b1) test positioning the electrode/module subassembly on the subject to establish the monitoring site;
      b2) making a record of the established monitoring site; and
      b3) removing the electrode release liner from the electrodes.
27. A monitor, comprising:
   one or more ECG electrodes;
   a securement component adapted to hold the one or more electrodes to the subject's body; and
   a host adapted to be removably connected to the one or more electrodes, the host including a sensor, a processor and an energy source.
28. A kit of components, comprising:
   one or more ECG electrodes;
   a securement component adapted to hold the one or more electrodes to the subject's body; and
   a host adapted to be removably connected to the one or more electrodes when the monitor is in use, the host including a sensor, a processor and an energy source.

## Claims

1. A suite of components for monitoring a subject, comprising:
one or more ECG electrodes;
a securement component adapted to hold the one or more electrodes to the subject's body; and
a host removably connected to or adapted to be removably connected to the one or more electrodes, the host including a sensor, a processor and an energy source.

2. The suite of components of claim 1 wherein the host comprises a power module which includes the energy source and a processor module which includes the processor.

3. The suite of components of claim 2 wherein the processor module also includes the sensor.

4. The suite of components of any preceding claim wherein the energy source is a battery and the power module includes a charging receiver.

5. The suite of components of any preceding claim wherein the host comprises:
a power module which includes a first housing comprised of a first housing base and a first housing cover, the first base and the first cover defining an interior of the first housing, and wherein the energy source is a battery residing in the interior space;
and
a processor module which includes a second housing comprised of a second housing base and a second housing cover, the second base and the second cover defining an interior of the second housing, the interior of the second housing containing:
a) a main circuit assembly,
b) an ON/OFF switch,
c) an accelerometer, and
d) a temperature sensing assembly.

6. The suite of components of claim 5 in which the power module and the processor module each include an electrode coupler whereby the power module can be coupled to at least one first ECG electrode by way of the power module electrode coupler(s) and the processor module can be coupled to at least one second ECG electrode by way of the processor module electrode coupler(s).

7. The suite of components of either claim 5 or claim 6 comprising a conduit having wiring extending therethrough for conveying electrical energy from the battery to the processor module to power components of the processor module.

8. The suite of components of any preceding claim wherein:
a) each electrode includes a snap assembly; and
b) the securement component is a pad having a bottom side and a top side, the bottom side including an adhesive, the pad also including a hole through which the snap assembly projects when the suite of components is being employed to monitor the subject.

9. The suite of components of claim 8 wherein the adhesive is one that adheres strongly enough to the subject's skin to resist detachment for approximately seven days and weakly enough to be intentionally removed from the subject's skin without damaging the subject's skin.

10. The suite of components of any preceding claim wherein:
a) each electrode includes a contact portion and a snap assembly extending from the contact portion;
b) the securement component is a pad having a bottom side and a top side, the bottom side including an adhesive, the pad also including a hole therethrough;
c) the host includes a base; and
d) when the suite of components is arranged to monitor the subject, the securement component resides between the base and the subject, a first portion of the securement component overlies the contact portion of the electrode, a second portion of the securement component sticks to the subject's skin, and the snap assembly projects into the host.

11. The suite of components of any preceding claim wherein, as initially encountered by an end user thereof, the host is separate from the one or more ECG electrodes.

12. The suite of components of claim 11 wherein:
the host includes exactly two modules as set forth below:
a first module adapted to be removably connected to M ECG electrodes, the first module including exactly M connection sites spatially distributed in a first connection site pattern and
a second module adapted to be removably connected to N ECG electrodes, the second module including exactly N connection sites spatially distributed in a second connection site pattern; and
the one or more ECG electrodes includes exactly N + M elecrodes.

13. The suite of components of either claim 11 or claim 12 wherein the one or more electrodes includes:
a first set of M electrodes which are spatially distributed or spatially distributable in a first electrode pattern which matches the first connection site pattern; and
a second set of N electrodes which are spatially distributed or spatially distributable in a second electrode pattern which matches the second connection site pattern.

14. The suite of components of claim 13 wherein N = M = 2 and the second connection site pattern is a replica of the first connection site pattern.

15. The suite of components of any one of claims 11 to 14 wherein:
the host includes exactly two modules as set forth below:
a first module which includes exactly two connection sites spaced apart by a first site spacing Ssi; and
a second module which includes exactly two connection sites spaced apart by a second site spacing S_{S2}; and
the one or more electrodes comprise:
a first set of exactly two electrodes which are spaced from each other by a first electrode spacing S_{E1} or are constrained to be spaceable from each other by no more than the first electrode spacing S_{E1} wherein S_{E1} = Ssi; and
a second set of exactly two electrodes which are spaced from each other by a second electrode spacing S_{E2} or are constrained to be spaceable from each other by no more than the second electrode spacing S_{E2} wherein S_{E2} = S_{S2}.
